(19) **Europäisches Patentamt / European Patent Office / Office européen des brevets**

(11) **EP 2 959 882 A1**

(12) **EUROPEAN PATENT APPLICATION**

(43) Date of publication:
**30.12.2015 Bulletin 2015/53**

(21) Application number: **14174759.2**

(22) Date of filing: **27.06.2014**

(51) Int Cl.:
*A61K 6/083* (2006.01)　　*A61L 24/00* (2006.01)
*A61K 6/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Stick Tech OY**
**20521 Turku (FI)**

(72) Inventors:
• **Lassila, Lippo**
**21630 Lielax (FI)**

• **Säilynoja, Eija**
**20660 Littoinen (FI)**
• **Vallittu, Pekka**
**21620 Kuusisto (FI)**

(74) Representative: **Suominen, Kaisa Liisa**
**Moosedog Oy**
**Rykmentintie 2B**
**20810 Turku (FI)**

(54) **A composite material and its uses**

(57)　The present invention relates to a composite material comprising matrix material in an amount of 20-40 weight-% of the total composition, filler material in an amount of 40-60 weight-% of the total composition, and inorganic fibre material an amount of 10-30 weight-% of the total composition, wherein the inorganic fibre material comprises short fibres having an average length of 80-300 $\mu$m and a diameter of 4-12 $\mu$m, in an amount of 20-80 weight-% of the total amount of fibre material and long fibres having an average length of 500-1300 $\mu$m and a diameter of 8-27 $\mu$m in an amount of 20-80 weight-% of the total amount of fibre material. The invention also relates to the use of this material in dentistry, especially as restorative filler material.

Fig. 3

**Description**

FIELD OF THE INVENTION

The present invention relates to a composite material comprising matrix material, filler material and inorganic fibre material according to preambles of enclosed independent claims. The invention also relates to uses of such composite materials.

BACKGROUND OF THE INVENTION

Various fibre-reinforced composite materials are known in the art. Despite the numerous materials known, there still exists a need for providing a composite material, especially for dental uses that has a suitable viscosity while at the same time leading to a product with good mechanical properties, such as strength.

In dental applications, composite materials are also used as restorative materials. In such applications, the composite material is surrounded by the natural tooth, i.e. by dentin and enamel. It would be preferable if the mechanical properties of the restorative material were either similar or better than those of enamel and dentin. The fracture toughness, $K_{IC}$ of human dentin has been discussed for example in "Fracture Toughness of Human Dentin, El Mowafy et al., J Dent Res 1986 65:677" and found to be approximately 3.08 MN.(m)$^{-1.5}$. The measurement method was comparable to that of standard ISO 20795-1:2008 Dentistry. Base polymers. Part 1: Denture base polymers.

OBJECT AND SUMMARY OF THE INVENTION

The object of the invention is to minimise or even totally eliminate the disadvantages existing in the prior art.

Therefore, the present invention relates to a composite material comprising matrix material in an amount of 20-40 weight-% of the total composition, filler material in an amount of 40-60 weight-% of the total composition, and inorganic fibre material an amount of 10-30 weight-% of the total composition, wherein the inorganic fibre material comprises short fibres having an average length of 80-300 $\mu$m and a diameter of 4-12 $\mu$m, in an amount of 20-80 weight-% of the total amount of fibre material and long fibres having an average length of 500-1300 $\mu$m and a diameter of 8-27 $\mu$m in an amount of 20-80 weight-% of the total amount of fibre material.

The invention also relates to uses of such composite material.

DETAILED DESCRIPTION OF THE INVENTION

The present invention relates to a composite material comprising

- matrix material in an amount of 20-40 weight-% of the total composition,
- filler material in an amount of 40-60 weight-% of the total composition, and
- inorganic fibre material an amount of 10-30 weight-% of the total composition, wherein the inorganic fibre material comprises

   - short fibres having an average length of 80-300 $\mu$m and a diameter of 4-12 $\mu$m, in an amount of 20-80 weight-% of the total amount of fibre material and
   - long fibres having an average length of 500-1300 $\mu$m and a diameter of 8-27 $\mu$m in an amount of 20-80 weight-% of the total amount of fibre material.

In this composite material, when it is applied to its intended final use, the matrix material is preferably at least partly in uncured form. By curing, it is meant either polymerisation or cross-linking or similar. The percentages in this description are weight-percentages (wt-%) of the total amount of components, unless otherwise stated. The composite material has improved fracture toughness and also improved handling properties, when compared to for example traditional dental restorative materials with similar fracture toughness properties.

The matrix material is preferably in its uncured form in the composite material before its application, and is cured once it is placed into its final position (such as in a dental cavity to be restored). The curing may be induced by light, heat or by a combination of an initiator/activator and light, or other wave energy such as UV or ultrasonic activation.

Since the matrix material is in its uncured form, the composite material may also be called a prepreg, i.e. a prepreg is an uncured composite, i.e. it contains all the components of the finished composite material, but the matrix material is still in monomer form, or when a crosslinkable matrix material is used, it its non-crosslinked form.

The composite material is also such that the fibres of the fibre material tend to orientate during their application, i.e. to align themselves. The composite material thus typically retains its original shape in the direction of the length of the fibres, even once the resin has been cured. This characteristic is especially beneficial, since it allows forming for example dental restorations where there are no gaps between the finished restoration material and the remaining tooth. The form stability of the composite material is at least partially based on the stiffness created by the fibre material and thus at least partially dependent on the fibre alignment in the composite material.

The filler material, typically in the form of particulate filler material, is typically selected such that it gives to the finished composite material its desired colour and radio-opacity. It typically also influences the shrinkage of the composite material compared to a composite material without any fillers, and increases its resistance to wear. The filler material is preferably inorganic filler material.

According to a preferred embodiment, the fibres of the fibre material are at least partly, sometimes even fully, silanated, i.e. surface treated such that at least a majority of their outer surface is covered with a silane component. It is also possible that the fibres become partly silanated, since the fibres are fully silanated when they are added to the composite material mixture, but as they break into shorter fibres during the mixing of the ingredients, the ends of the shorter fibres are no longer silanated. It is also possible that the fibre material comprises a mixture of silanated and non-silanated fibres. According to an embodiment, at least 90 % of the surfaces of the fibres in the fibre material are silanated. The silanation is such that it is compatible with the resin used as the matrix material, i.e. it contains active groups such as vinyl groups, acrylate groups, methacrylate groups or epoxy groups.

The fibres of the fibre material can thus be surface treated to enhance their bonding to the surrounding matrix material. The methods of surface treatment as well as the surface treating agents used may vary depending on the fibre material. The fibre material can be functionalised with e.g. hydroxyapatite, silanate, titanate, zirconate or aluminate. The fibre material may thus be both silanated and have another surface treatment.

According to one embodiment the short fibres and the long fibres may have different surface treatment. For example, the short fibres may have been silanated and functionalised with aluminate and the long fibres may have been only silanated. According to another embodiment the short fibres and the long fibres have the same surface treatment.

According to an embodiment, the inorganic fibre material is selected from the group consisting of inert glass fibres, bioactive glass fibres, sol-gel processed silica fibres, aluminium oxide based fibres, zirconia fibres, apatite fibres, quartz fibres and mixtures thereof. Preferably, the fibres are inert glass fibres.

According to one embodiment the long fibres and the short fibres may comprise different inorganic fibre material. According to another embodiment the long fibres and the short fibres may comprise the same inorganic fibre material.

The diameter of the short fibres used may vary from 4 μm up to 12 μm. The diameter can thus be for example from 4, 4.5, 5, 5.5, 6, 6.5, 7, 7.5, 8, 8.5, 9, 9.5, 10, 10.5, 11 or 11.5 μm up to 4.5, 5, 5.5, 6, 6.5, 7, 7.5, 8, 8.5, 9, 9.5, 10, 10.5, 11, 11.5 or 12 μm. According to one preferable embodiment the diameter of the short fibres used may be 4-8 μm.

The diameter of the long fibres used may vary from 8 μm up to 27 μm. The diameter can thus be for example from 8, 9, 10, 11, 13, 15, 16, 17, 19, 20, 22 or 23 μm up to 10, 11, 13, 15, 16, 18, 19, 20, 21, 23 or 25 μm. According to one preferable embodiment the diameter of the long fibres used may be 9-17 μm.

The diameter of the short and long fibres may be the same or different. According to one embodiment the long fibres have larger diameter than the short fibres.

The average length of the short fibres can be from 80 μm to 300 μm, such as from 80, 100, 120, 150, 170, 200, 220, 230 or 250 μm up to 120, 150, 180, 200, 210, 220, 230, 250, 280 or 300 μm. According to one embodiment of the invention the average length of the short fibres may be 100-300 μm. One preferable range of the average length of the short fibres in the finished composite material is 150-250 μm.

The average length of the long fibres can be from 500 μm to 1300 μm, such as from 500, 520, 550, 600, 650, 700, 800, 900, 1000, 1050 or 1200 μm up to 550, 600, 750, 800, 900, 1000, 100, 1200 or 1300 μm. One preferable range of the average length of the long fibres in the finished composite material is 700-900 μm. When fibres such as glass fibres are used, the long fibres are at the beginning of the manufacturing process of the composite material longer than in the final composite. Indeed, fibres such as glass fibres typically break during mixing of the components of the composition. According to the studies of the present inventors, the mixing method does not influence the final length of the fibres. Some practical examples for carrying out the invention are given below in the Experimental part, thus enabling a person skilled in the art to use the invention.

In context of the present application the average length of the fibres is determined as follows. The fibres are divided into a number of fractions with 0.1 mm intervals according to their length. The fibre lengths in each 0.1 mm interval are added together. The average fibre length is taken to be value where the lengths of the shorter fibres and longer fibres are deemed to be the same.

An interesting property of the present composite material is also its behaviour at fracture. Indeed, as is in shown below in the Experimental part, the composite material does not break in a sudden, catastrophic manner but rather in a slower, more controlled manner. This also leads to a more uniform and homogenous composite material, which behaviour is more predictable. Further, it is expected that in applications such as dental restorative materials, the composite material is more durable than previously known materials. The composite material is expected to be especially suitable for back teeth, where the occlusion forces are greater.

The total amount of inorganic fibre material is typically from 10 to 30 weight-% of the total composition. The total amount of fibre material of the final composite material can be for example from 10, 12, 15, 18, 20, 23 or 25 wt-% up to 12, 15, 20, 25 or 30 wt-% of the total weight of the composite material.

The amount of short fibres is typically 20-80 wt-% of the total amount of fibre material. The amount of short fibres of the final composite material can be for example from 20, 25, 30, 35, 40, 45, 50, 55, 60, 65, 70 or 75 wt-% up to 25, 30, 35, 40, 45, 50, 55, 60, 65, 70, 75 or 80 wt-% of the total amount of fibre material. One preferred range of the amount of the short fibres is 40-80 wt-% of the total amount of fibre material.

The amount of long fibres is typically 20-80 wt-% of the total amount of fibre material. The amount of long fibres of the final composite material can be for example from 20, 25, 30, 35, 40, 45, 50, 55, 60, 65, 70 or 75 wt-% up to 25, 30, 35, 40, 45, 50, 55, 60, 65, 70, 75 or 80 wt-% of the total amount of fibre material. One preferred range of the amount of the long fibres is 20-60 wt-% of the total amount of fibre material. There are preferably thus less long fibres than short fibres.

According to one preferable embodiment of the invention the amount of short fibres is as high as or higher than the amount of long fibres in the fibre material. The amount of the matrix material in the composite material can be 20-40 wt-% of the total weight of the composite material. According to one embodiment the amount of the matrix material in the composite material can be 30-40 wt-% of the total weight of the composite material. The amount of matrix material of the final composite material can be for example from 20, 22, 25, 27, 29, 30, 32 or 35 wt-% up to 22, 25, 27, 29, 32, 35 or 40 wt-% of the composite. The uncured matrix material, i.e. the matrix precursor used can be for example a monomer mixture comprising a solvent or a mixture of monomers without solvent. When the matrix material in its uncured form comprises a solvent, the solvent is typically removed from the composite material during curing.

The uncured matrix material may comprise monomers selected from the group consisting of methyl methacrylate, ethyl methacrylate, n-butyl methacrylate, isobutyl methacrylate, 2-ethylhexyl methacrylate, cyclohexyl methacrylate, isobornyl methacrylate, tetrahydrofurfuryl methacrylate, benzyl methacrylate, morpholinoethyl methacrylate, acrylic acid, diethylene glycol dimethacrylate, triethylene glycol dimethacrylate (TEGDMA), diurethane dimethacrylate, 2,2-bis(4-(2-hydroxy-3-methacryloxy)phenyl)propane (BisGMA), methacrylate functionalized dendrimers, other methacrylated hyperbranched oligomers and mixtures thereof. The matrix material may also be made of epoxy resins or rigid rod polymers such as poly(paraphenylene) based rigid rod polymers, epoxy resins being however not the preferred matrix materials. Acrylates and their various derivatives are part of the preferred matrix materials.

The matrix material may further be made of crosslinkable monomers or polymers such as ε-caprolactone, polycaprolactone, polylactides, polyhydroxyproline, and other biopolymers as well as polyamides, polyurethane, polyethylene, polypropylene, other polyolefins and polyvinyl chloride (PVC). The matrix material may naturally also consist of a mixture of a monomer(s) and a polymer(s).

According to an embodiment, the cured or uncured matrix material is a resin selected from the group consisting of methyl methacrylate, ethyl methacrylate, n-butyl methacrylate, isobutyl methacrylate, 2-ethylhexyl methacrylate, cyclohexyl methacrylate, isobornyl methacrylate, tetrahydrofurfuryl methacrylate, benzyl methacrylate, morpholinoethyl methacrylate, diethylene glycol dimethacrylate, triethylene glycol dimethacrylate, diurethane dimethacrylate, 2,2-bis(4-(2-hydroxy-3-methacryloxy)phenyl)propane, acrylic acid and mixtures thereof. One advantageous matrix material is a resin mixture of triethyleneglycol dimethacrylate and 2,2-bis(4-(2-hydroxy-3-methacryloxy)phenyl)propane.

The amount of particular filler material may be 40-60 wt-%. According to one embodiment the amount of particular filler material may be 40-50 wt-%. The amount of particular filler material of the final composite material can be for example from 40, 42, 45, 48, 50, 52, 55 or 58 wt-% up to 42, 45, 48, 50, 52, 55, 58 or 60 wt-% of the composite.

According to another embodiment, the particular filler material is selected from the group consisting of inert or bioactive or partially reactive glass ionomer fillers containing elements such as Si, Ca, P, Ba, Mg, K, Ti, F, Sr, Zn oxides or other compounds of said elements, colour pigments, inter ceramics, hydroxyl apatite (HA) or other Ca-phosphates, $Al_2O_3$, $ZrO_2$, Ag, zerogels, bioactive glasses or filler particles containing functional bioactive or therapeutically active molecules, antigens, antibiotics, disinfectants, radio-opaque materials, organic acids such as maleic acids, polyacrylic acid, or the like. The oxides can thus be for example silicon, calcium, phosphorus, barium, magnesium, potassium, titanium, fluorine, strontium or zinc oxide. By particles, it is meant also for example spheres and very short fibres (where the length of the fibre is at most two times its diameter), while being significantly shorter than the short fibres of the composite material, like whiskers i.e. having a length below < 50 μm. The particular filler material can be either bioactive or inert, such as bioactive glass, other additives, such as silicon (Si), calcium (Ca), phosphorus (P), barium (Ba), magnesium (Mg), potassium (K), sodium (Na) or titanium (Ti) oxides or fluorine (F) or other compounds of said elements, colour pigments, inert ceramics, hydroxyapatite or other calcium phosphates, xerogels, functionally bioactive or therapeutically active substances or radio opaque materials. The composite material may also further comprise growth factors, antigens, antibiotics, disinfectants, bone morphogenic proteins (BMPs), interferons, corticosteroids, bisphosphonates, cytostatics, anabolic hormones, vitamins, anti-inflammatory agents, antimicrobiotics and combinations and mixtures thereof. Preferably the particular filler material is inorganic filler material.

The particular filler material may also comprise either partly or fully glass ionomer powder. One type of glass ionomer powders are acid-soluble calcium fluoroaluminosilicate glass particles, which react with a reactive solvent and form a glass ionomer. The reactive solvent is typically poly(acrylic acid) (concentration between 40 to 50 %) or a copolymer or acrylic acid with itaconic, maleic, or tricarboxylic acids. The glass ionomer powder may have particles in the range of 15 to 50 $\mu$m. Typical percentages of the raw materials for glass ionomer powder are:

- silica 41.9 wt-%
- alumina 28.6 wt-%
- aluminium fluoride 1.6 wt-%
- calcium fluoride 15.7 wt-%
- sodium fluoride 9.3 wt-%
- aluminium phosphate 3.8 wt-%

The setting reactions in glass-ionomer materials are as follows (Nicolson, Croll. Quintessence Int 1997; 28:705-714).

1. Decomposition of the glass powder particles under the influence of the aqueous polyacid, i.e. reactive solvent, leading to the release of $Ca^{2+}$ and $Al^{3+}$ ions. The latter are probably released in the form of complex oxyanions containing several aluminium atoms, a structure that reflects the form they have occupied within the glass prior to acid attack.

2. Rapid reaction of the $Ca^{2+}$ ions with the polyacid chains, followed by slower reaction of $Al^{3+}$ species gradually released from the anionic complex. This reaction displaces water from some of the hydration sites and leads to some ionic crosslinking of the polyacid chains; both effects lead to insolubilisation of the polymer and stiffening of the material.

3. Gradual hydration of the inorganic fragments released in step 1, to yield a matrix of increasing strength, greater resistance to desiccation, and improved translucency.

Resin modified glass ionomers (RMGI) are conventional glass ionomers with addition of hydroxyethylmethacrylate (HEMA) and photoinitiators.

The diameter of the particles in the filler material (this being the largest diameter in case of irregular particles) may vary for example from 10 nm to 50 $\mu$m. The diameter can be for example from 10 nm, 50 nm, 100 nm, 500 nm, 1 $\mu$m, 5 $\mu$m, 10 $\mu$m, 25 $\mu$m or 40 $\mu$m up to 50 nm, 100 nm, 500 nm, 1 $\mu$m, 5 $\mu$m, 10 $\mu$m, 25 $\mu$m, 40 $\mu$m or 50 $\mu$m. Some preferred ranges are 100 nm - 40 $\mu$m.

The composite material can be used in various fields of dentistry, such as dental restoration material, post and core materials, endo-crown materials or for a pontic in fixed partial dentures. The composite material can also be used in other medical application such as orthopaedics as bone cement or in skull surgery. The composite material is especially suitable as a dental restorative material and it may be defined as flow-type restorative material. As will be demonstrated below in the Experimental part, the strength properties of the composite material are either similar or better than those of human dentine.

The invention also relates to the use of such composite material in a medical application. The medical application may be selected from the group consisting of dental applications and orthopaedic applications. The use may be as dental restorative material, as dental cement, for periodontal splinting, as bone cement or for maxillofacial prosthesis.

By the use of the present composite material as a dental composite cement, a high toughness for the finished composite material can be achieved. Some clinical applications are for example cementation of root canal posts or fixed dental prosthesis like metal-ceramic or zirconia framework. The composite material may be applied as paste in two syringes, wherein one of the syringes comprises a first chemical initiator (for example benzoyl peroxide) and the other syringe comprises a second chemical activator (for example a tertiary amine).

The composite material may also be used for direct periodontal splinting where the oriented fibres increase the fracture toughness, thus reinforcing mobile teeth and increasing stability during the healing period. Another application of the present invention is in a cement where a part of glass ionomer filler (GIC) is replaced with short fibres and the resulting filler material mixture is mixed with polyacrylic acid (such as maleic acid). The brittleness of the GIC can thus be decreased.

The present composite material may also be used in other biomedical applications, such as as bone cement, in maxillofacial prostheses and other orthopaedic applications. This results in improved handling properties of the composite material, which is especially beneficial in such applications where the cement penetration inside the bone morphology is important in order to create micromechanical retention/cementation.

The various embodiments and variations listed above apply mutatis mutandis to the use of a composite material according to this invention in medical applications.

EXPERIMENTAL PART

Composite material manufacturing

Raw materials:

- bisphenol A glycerolate dimethacrylate (bis-GMA; from Esschem)
- triethyleneglycol dimethacrylate (TEGDMA; from Aldrich)
- camphorquinone (QC) as initiator (from Aldrich)
- 2-(dimethylamino) ethyl dimethacrylate (DMAEMA) as co-initiator (from Aldrich)
- hydroquinone (HQ) as inhibitor(from Fluka)
- TEX 2400 - E-glass fibre (silanated, from Ahlstrom)
- E-glass fibres, length approximately 200 $\mu$m and diameter 6 $\mu$m (from Central Glass Fiber Co., Japan)
- barium silicate filler (UltraFine GM27884, from Schott)

Temperatures during the mixing were monitored by an infrared thermometer.

*1. Manufacturing of the resin mixture*

Weight HQ and TEGDMA to a container, the amounts being given below. Add the initiators QC and DMAEMA (amounts below) and continue mixing. Weight bis-GMA (amount below) to a separate mixing container and add TEGDMA. Mix by a centrifugal mixer (Hauschild Speedmixer DAC 400 FVZ) until fully mixed. The mixing speed and time depends on size of the mixing container used. NOTE: short mixing times may be required to prevent warming up of the mixture.

*Step 2 Addition of fibres and filler material*

Weight the filler material and fibres in the amounts indicated below and mix them. Weight the required amount of 6.5 mm long fibres. First mix the fibres to the resin mixture by a centrifugal mixer (Hauschild SpeedMixer DAC 400.1 VAC-P) without vacuum (15 sec at 2500 rpm speed). Finish mixing by spatula.
Add part of the prepared filler material to the resin/fibre containing mixing container and mix 15 seconds by 2500 rpm speed. Repeat until all the fillers are added.

Composite material compositions

Six different composite materials were manufactured, namely A, B, C, D, E and F.
In all the compositions, the amount of resin forming the matrix material and the relative amounts of its components were the same, i.e. approximately (the total being 100 %)

69.0 % of bis-GMA
29.0 % of TEGDMA
1.0 % of CQ
1.0 % of DMA
less than 0.1 % of HQ

The total amount of inorganic material (i.e. filler material and fibres) was also constant, 77 wt-% of the total composition of the composite in the compositions B, C, D and E. The total amount of inorganic material was 74 wt-% of the total composition of the composite in the compositions A and F, i.e. in the compositions not containing any short fibres or not containing any fibres at all. This reduced amount was due to the fact that 74 wt-% was the practical upper limit for manufacturing, when no short fibres were used.
The amounts of the components are given in Table 1.

Table 1

| Composition | A | B | C | D | E | F |
|---|---|---|---|---|---|---|
| Resin mixture (g) | 6.8 | 6.8 | 6.8 | 6.8 | 6.8 | 6.8 |
| Filler (g) | 15.0 | 15.0 | 15.0 | 15.0 | 15.0 | 19.4 |
| Short fibres (g) | 0 | 2.7 | 4.1 | 5.5 | 8.2 | 0 |

(continued)

| Composition | A | B | C | D | E | F |
|---|---|---|---|---|---|---|
| Long fibres (g) | 4.4 | 5.5 | 4.1 | 2.7 | 0 | 0 |

Fracture toughness

The fracture toughness was measured according to the standard ISO 20795-1:2008 Dentistry. Base polymers. Part 1: Denture base polymers.

For the specimen preparation a standard-dimensioned metal mould, with dimension of 2 mm x 5 mm x 25 mm, was used. The material was applied to the mould using an amalgam instrument. Then a thin razor blade was set on to the mould, in a way that it caused a pre-crack on to middle of the specimen. The depth of the crack was about 2.5 mm. When the blade was in its position, two strips of Mylar were set on both sides of it. By using the dental instrument, the material's surface was flattened out in a way that it was pressed against the blade to make the pre-crack's walls sharp.

On top of the Mylar-strip was set a microscope glass, thickness 1.0 mm. The material was polymerised for 3 x 40 s on one side, then the mould was turned around and the light curing was repeated. The hand-held light-curing device used was 3M Espe Elipar S10 (Serial nr 939123002122 230V/50/60Hz). The specimens were carefully removed and the sharp edges were grinned with 1200 grit SiC-paper. After a 24 h dry storage in a desiccator (ar 22 °C), the specimens were tested.

The fracture toughness test was performed with the mechanical testing machine Lloyd; Lloyd LRX30 7606.1259.98, Load cell 2500 N. Before testing, the height and the width was measured for each specimen. The dimensions were measured with a sliding gauge (from Mitutoyo (U.K) Ltd). The test was continued until the breakage of the specimen.

The following data information was taken into consideration:

- Date
- Sample Information
- Sample Reference
- Height (mm)
- Width (mm)
- Maximum Load (N)
- Kic
- F
- A/H=X (Attached part of the specimen is divided by the total height)

After the specimens were tested, they were photographed using a digital camera (Casio Exilim 20x Optical Zoom, Casio Computers Co LTD, China s/n 40012281A 76.1258.42).

With *Image J* - software, the fracture side of the specimen was measured from three different places and then the average length of the fracture side was calculated. This was done to all tested specimens. After measuring the lengths, the Toughness value was calculated again with an Excel-program.

For the result calculation, the result values from six (6) tested specimens from both groups were chosen. This number was chosen by taking into consideration the amount of prepared and successfully tested specimens. The values that were left out from the calculations were the ones that mostly differed from the average value or where the pre-crack point wasn't correctly done. The equations are as follows.

| | |
|---|---|
| Pre-crack | $a' = (3.0 \pm 0.2)$ mm |
| Crack length | $a$ (0.1 to 0.4 mm longer than a') |
| Width | $b_t = (4.0 \pm 0.2)$ mm |
| Height | $h_t = (8.0 \pm 0.2)$ mm |
| Span length | $l_t = (32.0 \pm 0.1)$ mm |

The maximum stress intensity factor, $K_{max}$, was calculated using the equation

$$Kmax = \frac{fP_{max}l_t}{(b_t h_t^{3/2})} \times \sqrt{10^{-3}} \, MPa \, m^{1/2}$$

where

*f* is a geometrical function dependent on x:

$$f(x) = 3x^{1/2} \, [1.99 - x(1-x)(2.15 - 3.93x + 2.7x^2]/[2(1+2x)(1-x)^{3/2}]$$

and

$$x = a/h_t$$

$P_{max}$ is the maximum load exerted on the specimen, in newtons,
$a, b_t, h_t$ and $l_t$ are expressed in millimetres, as explained above.

Fracture behaviour

In addition to the fracture toughness, the behaviour of the composite material at fracture point was also studied by measuring the required work from preload to maximum load (Ncm) as well as the required work from preload to maximum extension (Ncm). The difference [Work from preload to Maximum Extension (Ncm) - Work from preload to Maximum Load (Ncm)] gives the fracture behaviour of the composite material.

Flexural strength

The flexural strength was measured according to ISO 4049:2009 (E) standard (3-point bending test) (Dentistry - Polymer-based filling, restorative and luting materials)
Materials and equipment needed:

- protective cloves
- metallic sample mould (the preparation of mould is explained in the ISO 10477 and ISO 4049 standards)
- adhesive tape
- glass plate (microscope preparation glass plate)
- scissors
- calibrate; micrometer or equivalent measuring instrument (accuracy 0,01 mm)
- hand light curing unit
- universal mechanical testing machine (for example, Lloyd Instruments, Fareham, England)

*1) Sample preparation*

The test specimens were prepared in a metallic mould as explained in the ISO standard. First, the bottom side of the metallic mould was covered with adhesive tape to prevent material leaking during sample preparation. The mould was kept on a hard surface so that it was easy to push fibres tightly in to the groove with a glass plate.
Dimensions of the rectangular test specimens were height 2 mm, width 2 mm and length 25 mm. They were prepared as follows. Firstly, a metallic sample mould was filled with the material. Then a glass plate was placed on top of the filled mould and pressed tightly. The specimen was pre-light cured for 10 seconds and further light cured in light curing oven for 5 minutes. After curing, the specimen were removed from the mould and trimmed by gently abrasion with abrasive paper. A calibre was used to measure the size of the test specimen.
Samples were stored in a tight plastic bag for a minimum of 24 hours at room temperature before the measurements.

*2) Measurements*

Measure both the width and height of the test specimen from the middle of the specimen. These values are needed for the calculations. Place the specimen on the sample holder.

The test conditions are as follows:

- cross-head speed 1 mm/min
- span length 20 mm (span length is the distance between specimen supporters)

The samples are loaded with static force until fracture. The flexural strength was calculated by using the following equation (Alander P, Lassila LVJ, Vallittu PK. The span length and cross-sectional design affect values of strength. Dent Mater 2005; 21:347-353)

$$\sigma_f = (3Fl)/bh^2$$

wherein

$\sigma_f$ = flexural strength
F = load bearing capacity
l = the distance between the supporters (in millimetres)
b = width of the specimen (in millimetres)
h = height of the specimen (in millimetres)

The calculations were carried out by the equipment's software.

Volumetric shrinkage

The volumetric shrinkage was measured according to ISO 17304:2013 (En) standard (Dentistry - Polymerization shrinkage: Method for determination of polymerization shrinkage of polymer-based restorative materials). The only difference to the standard procedure was that a sample weight of 0.2 g was used instead of 1 g, which is originally defined in the standard. The standard specifies requirements for dental polymer-based restorative materials supplied in a form suitable for mechanical mixing, hand-mixing, or intra-oral and extra-oral external energy activation, and intended for use primarily for the direct or indirect restoration of cavities in the teeth and for luting.

The standard method is based on the buoyancy method (Archimedes' principle). In this method, the material densities before and after polymerization are recorded. It is important to measure the temperature exactly, so that Archimedes' buoyancy principle can be reliably applied.

Individual densities are calculated by:

$$\rho_x = \frac{m1 x \rho_0}{m1 - m2}$$

wherein

px = is the density of the sample, in g per ml;
m1 = is the mass of the sample in position 1 an air, in g;
m2 = is the mass of the sample in position 2 in the buoyancy medium, in g; and
$\rho_0$ = is the density of the buoyancy medium, in g per ml, at the respective measuring temperature.

Polymerization shrinkage is calculated by:

$$S = \left(\frac{\overline{\rho_c} - \overline{\rho_u}}{\overline{\rho_c}}\right) \times 100$$

where

S = is the polymerization shrinkage, in percentage term;
$\overline{\rho_u}$ = is the average value of density of the unpolymerized sample, in g per ml; and
$\rho_c$ = is the average value of the density of the polymerized sample, in g per ml.

The obtained results are shown in Table 2.

Table 2

| Sample | Volumetric shrinkage [%] |
|---|---|
| Composition B | 3.77 |
| Composition C | 3.45 |
| Composition D | 4.54 |
| Composition E | 4.95 |

From Table 2 it can be seen that the long fibres had a positive effect on the volumetric shrinkage. All the obtained values were acceptable, but it can be seen that the long fibres in the composite material reduce the volumetric shrinkage (compositions B, C, D) when compared to composite without long fibres (composition E).

Fibre length

*Method*

A small portion of the material (approximately 0.1 g) was taken and set on a glass ware. Then 2 ml of tetrahydrofuran (THF, Riedel de Haen, Puriss 99.9%, Lot; 404750) was set onto the glass using a Pasteur-pipette. A spatula was then used to stir the portion in THF, so that the extraction process would begin. When it was seen that the material had started to extract, the THF was removed with another Pasteur-pipette, and fresh THF was set again onto the glass. This cycle was continued until the THF stayed clear even though it was stirred with the spatula on the glass and the fibres could be seen visually.
After the last added THF had vaporised and the fibres had dried, they were transferred on a microscope glass plate. Carefully a few drops of ethanol was added onto the glass to separate the fibres (ethanol was used because keeps the glass clear, when the final specimens are made). After the ethanol had been added, a thinner glass plate was taken (dimension 20 x 20 mm) and moved sideways against the microscope glass to make the fibres attach to it. The glass plates were then left to dry.
After the glass plates had dried, they were photographed with a stereomicroscope, Wild Herbrugg 76.3.35.34 Art NR 473849 Type MDG 17 Switzerland. The magnification used was x 6.5. The stereomicroscope was calibrated before the pictures were taken.
The fibre lengths were measured from the pictures with Image-J programme. The programme was scaled with a picture taken of a Vickers-ruler with same magnification. The scale was set for 1 mm. For each group, the lengths of 400 fibres were measured.

Extrusion force - procedure

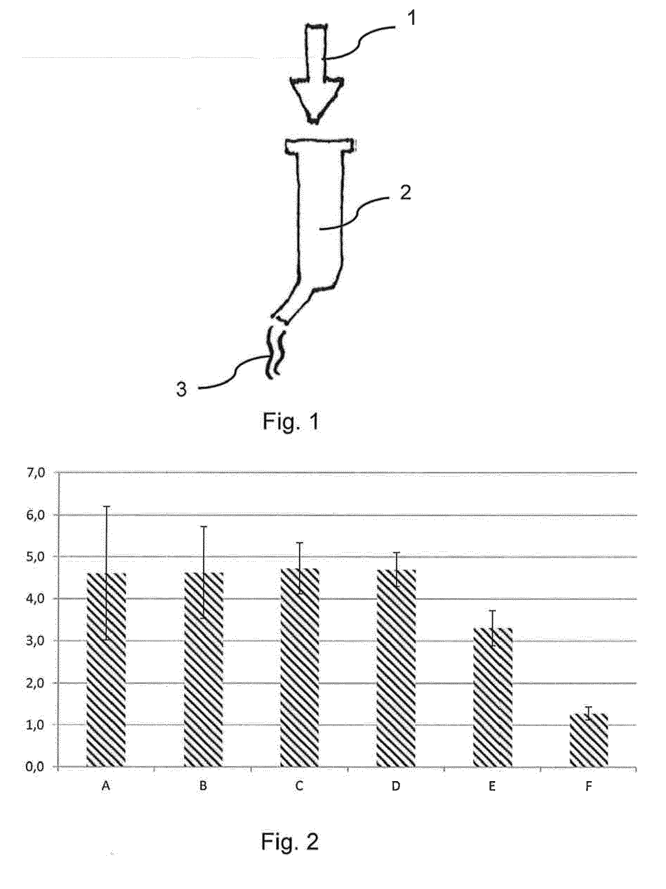

Extrusion force is the force required to extract the material from a single dose tip. The measurements were made with a mechanical testing device (Universal Testing Machine, Lloyd LRX Plus) by measuring the strength/force needed during pressing the plunger downwards. The measurement set up is shown in Figure 1, where the reference number 1 shows the direction of the force, reference number 2 the testing device (single dose tip) and reference number 3 denotes the material flow out from the device.

*Results*

The measurements of fibre length for compositions B to E gave the results shown in Table 3, as total fibre length at corresponding fraction, in mm.

Table 3

| Fibre length fractions | B | C | D | E |
|---|---|---|---|---|
| < 0.1 | 0.91 | 1.87 | 4.4 | 2.4 |
| 0.1 | 13.2 | 21.9 | 15.8 | 25.9 |
| 0.2 | 21.8 | 17.2 | 34.0 | 28 |
| 0.3 | 18.3 | 11.7 | 29.1 | 9.3 |
| 0.4 | 26 | 16 | 18 | 0.4 |
| 0.5 | 20 | 25 | 13 | 1.6 |
| 0.6 | 18.6 | 29.4 | 16.8 | |
| 0.7 | 16.1 | 37.1 | 19.6 | |
| 0.8 | 22.4 | 32.8 | 16.8 | |
| 0.9 | 10.8 | 36.9 | 13.5 | |
| 1.0 | 15 | 19 | 11 | |
| 1.1 | 2.2 | 9.9 | 9.9 | |
| 1.2 | | 10.8 | 7.2 | |
| 1.3 | 1.3 | 7.8 | 1.3 | |
| 1.4 | | | 5.6 | |
| 1.5 | | 4.5 | 3 | |
| 1.6 | | 3.2 | 4.8 | |
| 1.7 | | 1.7 | | |
| 1.8 | 1.8 | | | |
| 1.9 | | 1.8 | 8 | |
| ≥ 2.0 | | | 4 | |

Crown Compression Strength Test

The aim of the test was to perform a crown compression strength test for a composite matrix according to the present invention and to compare it to 1) a commercial composite material comprising fibres with unimodal fibre length distribution and 2) commercial composite material without fibres.

The model tooth used was upper incisor.

*Test Method*

The test was started by preparing a mould, made out of silicone. A crown was built over a zirconia-model by using a commercial composite without fibres. After the material was lightcured it was pressed into the Memosil-silicone and left to harden.

The test crowns for the crown compression strength test were prepared by filling the crown area of the prepared silicone mould with a material to be tested and the zirconia-model was pressed into the silicone mould.

Material in the mould was lightcured 2 x 20 s through the silicone. Then the test crown was removed from the mould and the lightcuring was continued for 4 x 20 s, i.e. 20 s to each side of the crown.

After the lightcuring each test crown was left to postpolymerize for 10 min before the crown compression strength test was carried out. The postpolymerisation time was measured with a timer.

The crown compression strength test was performed with a mechanical testing device Lloyd LR 30k Plus, Sensor 2500 N (Ametek Inc.).

Test settings were as follows:

Direction: Compression;

Preload: 5N
Preload speed: 5mm/min
Extension rate: 1 mm/min
Limits: Extension 8mm

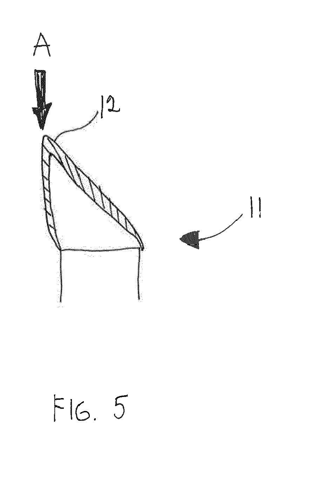

Figure 5 gives a schematical view of the test procedure. On the test the crown 11 to be tested was loaded to the crown part 12 until the breakage point was reached. The applied compression force and its direction are presented with an arrow A. Three measurements were made for each composite material.

The results of crown compression strength test are shown in Table 4. The results are given as an average of the three tests.

Table 4

| Crown Material | Load at Max. Load [N] | st. Dev. | Work from preload to Break [Ncm] | st. Dev. |
|---|---|---|---|---|
| Composite material with short & long fibres | 934.0 | 85.7 | 23.6 | 6.3 |
| Commercial composite material with unimodal fibre distribution | 770.9 | 30.5 | 14.0 | 1.2 |
| Commericial composite without fibres | 373.8 | 19.7 | 4.3 | 0.1 |

It can be seen from Table 4 that the crown made from composite material according to the invention, i.e. with both short and long fibres, was able to resist a higher load than the crowns made from commercial composite material. Similarly the work needed for preload to break was higher for the composite material according to the present invention.

Some results are shown in Figures 2 to 4, wherein Figure 2 illustrates the results of measurements of fracture toughness, Figure 3 the results of measurement of fracture behaviour and Figure 4 the results of the measurements of flexural strength.

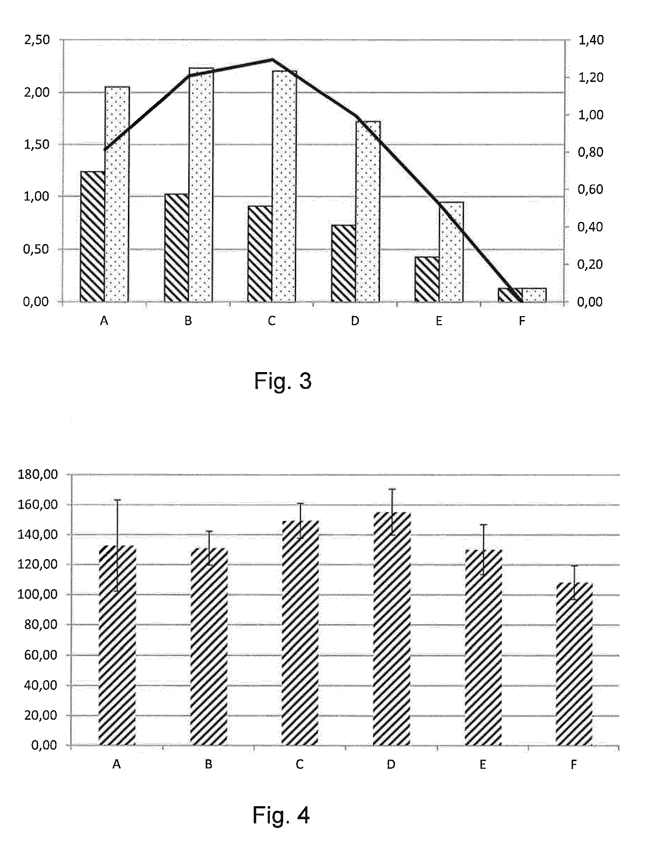

Indeed, Figure 2 shows the values of $K_{max}$ as well as the standard deviation. In Figure 3, the work from preload to maximum load (Ncm) is shown as the left column in each pair, the work from preload to maximum extension (Ncm) as the right column in each pair and their difference (work of fracture) is shown as the line. As can be seen, the inclusion of longer fibres increases the work of fracture, i.e. the force required for complete fracture of the material. This indicates that the material would be safer to use as there would not be immediate fractures.

In Figure 4, the maximum flexural strength, in MPa, is given for each composition, as well as the standard deviation. As can be seen from all these results, the combination of two different fractions of fibres (i.e. of two fractions of fibres having different length), the amount of reinforcing, longer fibres can be increased without significant deterioration of the handling properties of the material. One notable consequence of the use of the present composite material compared to compositions A and F is that even if in some compositions the mechanical properties are similar, the standard deviation decreases significantly, meaning that the products are more homogeneous. This leads to a better usability of the material.

Moreover, the required extrusion strength was also measured for the compositions B to E, while the matrix material was still in uncured form, and was found to be 17 N for composition B, 14 N for composition C, 8 N for composition D and 3 N for composition E. The extrusion strength also indicates the force required to extract the composition from a syringe, i.e. the lower the force, the easier the material is to handle.

Based on these results, it can be seen that when using only short fibres in the composition, the material has good handling properties but its mechanical properties are not as good as for materials comprising also longer fibres. On the other hand, if only long fibres are used, the handling properties as well as the homogeneity of the material are reduced. This is seen for example in the increased standard deviation of the results (as shown in the Figures 2 and 4).

**Claims**

1.  A composite material comprising

    - matrix material in an amount of 20-40 weight-% of the total composition,
    - filler material in an amount of 40-60 weight-% of the total composition, and
    - inorganic fibre material an amount of 10-30 weight-% of the total composition, wherein the inorganic fibre material comprises

- short fibres having an average length of 80-300 $\mu$m and a diameter of 4-12 $\mu$m, in an amount of 20-80 weight-% of the total amount of fibre material and
- long fibres having an average length of 500-1300 $\mu$m and a diameter of 8-27 $\mu$m in an amount of 20-80 weight-% of the total amount of fibre material.

2. A composite material according to claim 1, **characterised in that** the average length of the short fibres is 150-250 $\mu$m.

3. A composite material according to claim 1 or 2, **characterised in that** the average length of the long fibres is 700-900 $\mu$m.

4. A composite material according to any of the previous claims, **characterised in that** the amount of short fibres is 40-80 weight-% of the total amount of fibre material and the amount of long fibres is 20-60 weight-% of the total amount of fibre material.

5. A composite material according to any of the previous claims, **characterised in that** in the fibre material the amount of short fibres is as high as or higher than the amount of long fibres.

6. A composite material according to any of the previous claims, **characterised in that** the diameter of the short fibres is 4-8 $\mu$m.

7. A composite material according to any of the previous claims, **characterised in that** the diameter of the long fibres is 9-17 $\mu$m.

8. A composite material according to any of the previous claims, **characterised in that** the inorganic fibre material is selected from the group consisting of inert glass fibres, bioactive glass fibres, sol-gel processed silica fibres, aluminium oxide based fibres, zirconia fibres, apatite fibres, quartz fibres and mixtures thereof.

9. A composite material according to claim 8, **characterised in that** the fibres of the inorganic fibre material are inert glass fibres.

10. A composite material according to any of the previous claims, **characterised in that** the matrix material is a resin selected from the group consisting of methyl methacrylate, ethyl methacrylate, n-butyl methacrylate, isobutyl methacrylate, 2-ethylhexyl methacrylate, cyclohexyl methacrylate, isobornyl methacrylate, tetrahydrofurfuryl methacrylate, benzyl methacrylate, morpholinoethyl methacrylate, diethylene glycol dimethacrylate, triethylene glycol dimethacrylate, diurethane dimethacrylate, 2,2-bis(4-(2-hydroxy-3-methacryloxy)phenyl)propane, acrylic acid and mixtures thereof.

11. A composite material according to claim 10, **characterised in that** the matrix material is a resin mixture of triethyleneglycol dimethacrylate and 2,2-bis(4-(2-hydroxy-3-methacryloxy)phenyl)propane.

12. A composite material according to any of the previous claims, **characterised in that** the filler material is selected from the group consisting of inert or bioactive or partially reactive glass ionomer fillers containing elements such as Si, Ca, P, Ba, Mg, K, Ti, F, Sr, Zn oxides or other compounds of said elements, colour pigments, inter ceramics, hydroxyl apatite or other Ca-phosphates, $Al_2O_3$, $ZrO_2$, Ag, zerogels, bioactive glasses or filler particles containing functional bioactive or therapeutically active molecules, antigens, antibiotics, disinfectants and/or radio-opaque materials.

13. Use of a composite material according to any of the claims 1-12 in a medical application.

14. Use according to claim 13, **characterised in that** said medical application is selected from the group consisting of dental applications and orthopaedic applications.

15. Use according to claim 14, **characterised in that** said use is as dental restorative material, as dental cement, for periodontal splinting, as bone cement or for maxillofacial prosthesis.

Fig. 1

Fig. 2

Fig. 3

Fig. 4

A

12

11

FIG. 5

Europäisches
Patentamt

European
Patent Office

Office européen
des brevets

**EUROPEAN SEARCH REPORT**

Application Number

EP 14 17 4759

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 0 345 581 A2 (BAYER AG [DE]) 13 December 1989 (1989-12-13) * abstract * * page 2, line 31 - line 49 * * page 3, line 55 - page 4, line 23 * * claims * | 1-15 | INV. A61K6/083 A61L24/00 A61K6/00 |
| Y | US 5 266 609 A (HALL NEIL R [AU] ET AL) 30 November 1993 (1993-11-30) * column 2, line 32 - column 3, line 47 * * claims; examples * | 1-10, 12-15 | |
| Y | US 6 270 348 B1 (RICHARD PETERSON) 7 August 2001 (2001-08-07) * column 1, line 65 - line 45 * * column 4, line 65 - column 5, line 30 * * Formula II, Formula III; table 3 * * column 7, line 32 - line 39 * | 1,3, 8-11, 13-15 | |
| Y | US 4 107 845 A (LEE JR HENRY L ET AL) 22 August 1978 (1978-08-22) * column 2, line 37 - line 43 * * column 3, line 1 - line 59 * * column 4, line 43 - column 6, line 28 * * column 12, line 14 - line 25 * * claims; examples * | 1-15 | TECHNICAL FIELDS SEARCHED (IPC) A61K C08J |
| Y | WO 2008/000917 A1 (STICK TECH OY [FI]; LASSILA LIPPO V J [FI]; VALLITTU PEKKA [FI]; GAROU) 3 January 2008 (2008-01-03) * page 3, line 11 - line 18 * * page 17, line 11 - line 12 * | 1 | |

-/--

The present search report has been drawn up for all claims

| Place of search | Date of completion of the search | Examiner |
|---|---|---|
| Berlin | 8 December 2014 | Pelli Wablat, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**EP 2 959 882 A1**

Europäisches
Patentamt
European
Patent Office
Office européen
des brevets

# EUROPEAN SEARCH REPORT

Application Number

EP 14 17 4759

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2013/000947 A1 (STICK TECH OY [FI]; LASSILA LIPPO [FI]; VALLITTU PEKKA [FI]; KEULEMANS) 3 January 2013 (2013-01-03)<br>* page 5, line 4 - line 8 *<br>* page 6, line 19 - page 7, line 3 *<br>* page 8, line 8 - line 26 *<br>* page 13, line 19 - line 28; figure 10 *<br>* claims; examples 1,2 *<br>----- | 1-15 | |
| | | | **TECHNICAL FIELDS SEARCHED (IPC)** |

The present search report has been drawn up for all claims

| Place of search | Date of completion of the search | Examiner |
|---|---|---|
| Berlin | 8 December 2014 | Pelli Wablat, B |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

18

## ANNEX TO THE EUROPEAN SEARCH REPORT
## ON EUROPEAN PATENT APPLICATION NO.

EP 14 17 4759

This annex lists the patent family members relating to the patent documents cited in the above-mentioned European search report.
The members are as contained in the European Patent Office EDP file on
The European Patent Office is in no way liable for these particulars which are merely given for the purpose of information.

08-12-2014

| Patent document cited in search report | | Publication date | Patent family member(s) | | Publication date |
|---|---|---|---|---|---|
| EP 0345581 | A2 | 13-12-1989 | DE | 3819777 A1 | 21-12-1989 |
| | | | DK | 282889 A | 11-12-1989 |
| | | | EP | 0345581 A2 | 13-12-1989 |
| | | | JP | H0238402 A | 07-02-1990 |
| | | | PT | 90715 A | 29-12-1989 |
| US 5266609 | A | 30-11-1993 | NONE | | |
| US 6270348 | B1 | 07-08-2001 | NONE | | |
| US 4107845 | A | 22-08-1978 | NONE | | |
| WO 2008000917 | A1 | 03-01-2008 | BR | PI0713512 A2 | 31-01-2012 |
| | | | CA | 2656864 A1 | 03-01-2008 |
| | | | EP | 2034948 A1 | 18-03-2009 |
| | | | FI | 20075075 A | 06-08-2008 |
| | | | HK | 1132661 A1 | 13-12-2013 |
| | | | JP | 5345054 B2 | 20-11-2013 |
| | | | JP | 2009541568 A | 26-11-2009 |
| | | | KR | 20090024816 A | 09-03-2009 |
| | | | RU | 2009102991 A | 10-08-2010 |
| | | | US | 2009258965 A1 | 15-10-2009 |
| | | | WO | 2008000917 A1 | 03-01-2008 |
| WO 2013000947 | A1 | 03-01-2013 | EP | 2540481 A1 | 02-01-2013 |
| | | | WO | 2013000947 A1 | 03-01-2013 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 939123002122 A

**Non-patent literature cited in the description**

- **EI MOWAFY et al.** Fracture Toughness of Human Dentin. *J Dent Res,* 1986, vol. 65, 677
- **NICOLSON.** *Croll. Quintessence Int,* 1997, vol. 28, 705-714
- **ALANDER P ; LASSILA LVJ ; VALLITTU PK.** The span length and cross-sectional design affect values of strength. *Dent Mater,* 2005, vol. 21, 347-353